# EUROPEAN PATENT SPECIFICATION

(11) **EP 4 124 292 B1**
(45) Date of publication and mention of the grant of the patent: **25.09.2024**
(21) Application number: 21188487.9
(22) Date of filing: 29.07.2021
(51) Int. Cl.: A61B 5/097, A61B 5/087, G01F 1/66, B01D 46/10

(54) **BREATHING TUBE ARRANGEMENT FOR A LUNG FUNCTION DIAGNOSTICS DEVICE COMPRISING A DISTAL FILTER ELEMENT**
ATEMSCHLAUCHANORDNUNG FÜR EIN LUNGENFUNKTIONSDIAGNOSEGERÄT MIT EINEM DISTALEN FILTERELEMENT
AGENCEMENT DE TUBE RESPIRATOIRE POUR DISPOSITIF DE DIAGNOSTIC DE LA FONCTION PULMONAIRE COMPRENANT UN ÉLÉMENT DE FILTRATION DISTAL

(43) Date of publication of application: 01.02.2023
(73) Proprietor: ndd Medizintechnik AG, 8005 Zürich (CH)
(72) Inventor: DANERS, Felix, 8200 Schaffhausen (CH); FRÖHLICHER, Fabian, 6003 Luzern (CH); RENGGLI, David, 6015 Luzern (CH); BUESS, Christian, 8810 Horgen (CH)
(74) Representative: Maikowski & Ninnemann Patentanwälte Partnerschaft mbB

(56) References cited:
- US-A- 5 647 370
- US-A1- 2014 180 156
- US-A1- 2020 359 936

## Description

The present invention relates to a breathing tube arrangement according to claim 1 and to a lung function diagnostics device comprising such a breathing tube arrangement according to claim 12.

A plurality of different breathing tubes for use in lung function diagnostics is known from prior art. These breathing tubes serve for guiding respiratory air to be analyzed from a subject through a lung function diagnostics device.

EP 3 017 760 A1 describes such a breathing tube for use in ultrasonic flow measurement systems for determining the volume flow and/or the molar mass of the respiratory gas of a human or animal subject. This European patent application also describes the general possibility to provide the breathing tube with a filter element. Such element is used to reduce the risk of contaminations of the lung function diagnostics device. In addition, it also reduces the risk of contaminations of the environment considering the general possibility that a subject yet unperceivably suffers from a respiratory disease, such as COVID-19, at the time of the lung function analysis. Such a filter element also reduces the risk of inhaling contaminated air by a subject during the analysis of the subject's respiratory air. Consequently, an infection of the subject and the medical staff assisting the subject in performing the lung function analysis is significantly reduced when providing the used breathing tube with a filter element. Furthermore, the use of such a filter element has been made compulsory in many countries of the world during the COVID-19 pandemic.

However, the use of a filter element together with a breathing tube often significantly increases the breathing resistance for the subject. An increased breathing resistance accounts for a less comfortable lung function analysis. In addition, an increased breathing resistance results in a change of the measured lung function parameters. Therefore, the breathing resistance should be as low as possible. The joined guidelines of the American Thoracic Society (ATS) and the European Respiratory Society (ERS) (B.L. Graham et al: Standardization of Spirometry 2019 Update, Am J Respir Crit Care Med, Vol 200, Issue 8, pp e70-e88, Oct 15, 2019), as well as the standard ISO 26782:2009 require that the breathing resistance of a spirometer be smaller than 1.5 cm H₂O/L/s at a flow in a range of from 0 to 14 L/s.

Breathing tube arrangements known from prior art typically comprise a breathing tube and a filter element located between a breathing tube analysis zone and a mouthpiece of the breathing tube. Expressed in other words, the filter element is proximally positioned. This proximal position ensures that only filtered air flows through a flow sensor so as to avoid a contamination of the flow sensor. However, since no 100 % filtration of the air is possible, the flow sensor needs nonetheless to be cleaned in regular intervals. To achieve a sufficiently small breathing resistance, the filter element typically comprises a filter disc with a quite big diameter.

Further breathing tube arrangements according to the state of the art are for instance described in US 2020/0359936 A1, US 2014/0180156 A1, and US 5,647,370 A.

It is an object of the present invention to provide a breathing tube arrangement with a filter element that efficiently reduces the infection risk for a subject and for medical staff and that has smaller breathing resistance than filter-equipped breathing tubes known from prior art.

This object is achieved with a breathing tube arrangement for a lung function diagnostics device having the claim elements of claim 1. Such a breathing tube arrangement comprises a breathing tube that defines a guiding path for breathing air to be analyzed by a lung function diagnostics device. The breathing tube has a proximal end, a distal end and an analysis zone located between the proximal end and the distal end. The analysis zone is intended to be inserted into a lung function diagnostics device. Typically, the analysis zone comprises one window on each side to allow an interaction between the breathing air flowing through the analysis zone and an analytic medium of the lung function diagnostics device. Particularly appropriate analytic media are electromagnetic waves, such as infrared radiation, and/or ultrasonic waves.

The proximal end of the breathing tube is designed to face a subject whose lung function is to be analyzed. It furthermore serves for providing gas/breathing air to be inspired by the subject and for receiving exhaled breathing air from the subject. The analysis zone serves for allowing an analysis of the gas or breathing air flowing through the breathing tube. If the lung function diagnostics device that receives the breathing tube is an ultrasonic spirometer, the analysis of the breathing air is done by ultrasonic waves.

The distal end of the breathing tube serves for releasing analyzed breathing air to an environment of the breathing tube or to devices arranged downstream to the breathing tube (more distally than the breathing tube).

The breathing tube arrangement comprises a filter element connected to the distal end of the breathing tube. Breathing air flowing from the breathing tube through the distal end of the breathing tube towards an environment of the breathing tube has then to pass the filter element.

Thus, in contrast to prior art solutions, the breathing tube arrangement does not comprise a proximally positioned filter element, but rather a distally positioned filter element. A proximally positioned filter element is positioned between a mouthpiece (if any) of the breathing tube and an analysis zone of the breathing tube. Therefore, it typically exhibits a rather symmetric design and comprises two cylindrical or polygonal connectors with comparatively small diameters at both ends of the filter element. These connectors serve on the one hand for a connection to the mouthpiece and on the other hand for a connection to the analysis zone of the breathing tube. Due to their comparatively small diameter and due to an expansion of the breathing gas over the filter element and a subsequent contraction of the breathing gas when entering the connector downstream the filter element, these arrangements cause a significant increase in the breathing resistance of the breathing tube.

By connecting the filter element to the distal end of the breathing tube, the design of the filter element can be much more freely chosen. In particular, it is no longer necessary to design the distally positioned filter element in a symmetric manner. Rather, only a single connector is necessary to connect the filter element to the breathing tube. No small-diameter connector is any longer necessary at the distal end of the filter element. Therefore, the distal position of the filter element allows an efficient and reliable filtration of air being exhaled from the subject through the breathing tube towards an environment as well as of air being inhaled by the subject through the breathing tube.

The filter element comprises a filter material, a breathing air releasing region arranged distally of the filter material, and a diffusor zone arranged proximally of the filter material. The diffusor zone serves for significantly reducing the breathing resistance of the whole system (i.e., of the breathing tube arrangement comprising the breathing tube and the filter element). The breathing resistance can - depending on the concrete design of the filter element - even be lower than that of a breathing tube without any filter element.

The diffusor zone comprises a proximal end with a first proximal cross-sectional area and a distal end with a first distal cross-sectional area. The first distal cross-sectional area is bigger than the first proximal cross-sectional area. By such a design of the filter element, it is guaranteed that exhaled breathing air can exit from the breathing tube arrangement through the filter element along a flowing path having at least section-wise an increasing diameter, in particular at least section-wise a continuously increasing diameter. Expressed in other words, the diffusor zone comprises an expansion from its proximal end towards its distal end. Such an expansion is particularly appropriate to reduce the breathing resistance of the filter element and to allow a particularly easy release of breathing gas from the breathing tube through the filter element towards an environment of the filter tube arrangement.

The proximal end of the diffusor zone is connected to the distal end of the breathing tube. Due to this connection, breathing air that flows from the breathing tube through the distal end of the breathing tube towards an environment of the breathing tube arrangement has to pass the filter element. Likewise, air or gas to be inspired by a subject first has to pass the filter element before entering the breathing tube and reaching the subject.

The breathing air releasing region of the filter element has a second distal cross-sectional area. This second distal cross-sectional area is bigger than the first proximal cross-sectional area (of the diffusor zone) and at least as big as the first distal cross-sectional area (of the diffusor zone). It can even be bigger than the first distal cross-sectional area. A ratio between the first proximal cross-sectional area and the second distal cross-sectional area lies in a range of from 1:2 to 1:20, in particular of from 1:2.5 to 1:19, in particular of from 1:3 to 1:18, in particular of from 1:3.5 to 1:17, in particular of from 1:4 to 1:16, in particular of from 1:4.5 to 1:15, in particular of from 1:5 to 1:14, in particular of from 1:6 to 1:13, in particular of from 1:7 to 1:12, in particular of from 1:8 to 1:11, in particular of from 1:9 to 1:10.

Such an arrangement of the diffusor zone and the breathing air releasing region is particularly appropriate for reducing the breathing resistance of the whole arrangement.

As already explained above, the analysis zone of the breathing tube typically comprises two windows at opposite sides of the analysis zone. These windows are typically covered with a mesh so that a laminar flow within the breathing tube can be established. Without covering the windows with a mesh, vortices in the flow of gas flowing through the breathing tube may occur. Such vortices can impart the quality of the gas analysis.

It should be noted that the presently claimed and described breathing tube arrangement is not only appropriate for an analysis of exhaled breathing air flowing from the proximal end of the breathing tube through the analysis zone and exiting the breathing tube arrangement through the filter. Rather, it also appropriate for an analysis of inhaled breathing air entering the breathing tube through the distally positioned filter element and flowing along the guiding path from the distal end of the breathing tube towards the proximal end of the breathing tube. The distally arranged filter element does not limit the possible applications of the breathing tube arrangement in any way.

As explained above, the filter element is connected with its proximal end to the distal end of the breathing tube. In an embodiment, a distal end of the breathing air releasing region is designed without a connector for connecting further elements of a breathing gas flow path to the breathing gas releasing region. Such an asymmetric design of the filter element results in a significant material reduction of the filter element. Thus, the filter element can be produced in a more cost-efficient and environmentally friendly manner than filter elements known from prior art. Such a design also results in a much smaller dead space of the filter element and therewith in a smaller overall dead space of the breathing tube arrangement. The smaller the dead space of the breathing tube arrangement, the more possible applications exist for the breathing tube arrangement (e.g., it can also be used for a breathing gas analysis of children). Furthermore, a smaller dead space generally facilitates the use of the breathing tube arrangement for a subject. This asymmetric design with a single connector only being present at the proximal end (or inlet) of the filter element significantly reduces the breathing resistance of the filter element and thus of the whole breathing tube arrangement since the exhaled air does no longer need to pass a small-diameter tube after the filter element, but can simply exit from the breathing tube arrangement over the whole area of a filter contained in the filter element.

In an embodiment, the reduction of the breathing resistance achieved by implementing a diffusor zone in the filtering element can be exploited to increase the filter efficiency. Thus, it is possible to use a filter material having a higher flow drop than filter materials used according to prior art (e.g., due to a higher thickness of the filter material). The increased breathing resistance of such a filter material is over-compensated (or at least fully compensated) by the decreased breathing resistance due to the diffusor zone. Thus, the diffusor zone enables a higher filter efficiency while maintaining or reducing the breathing resistance with respect to common filter elements.

In an embodiment, the cross section of the inlet of the filter element is smaller than a cross section of the outlet. As laid out above, an outlet of the filter element having a big cross section serves for a significant reduction of the breathing resistance of the filter element and thus of the overall breathing tube arrangement.

In an embodiment, the second distal cross-sectional area (i.e., the cross-sectional area of the breathing gas releasing region that typically roughly corresponds to the cross-sectional area of the filter material through which gas flowing through the breathing tube arrangement can pass) has a size lying in a range of from 30 cm² to 200 cm², in particular of from 35 cm² to 190 cm², in particular of from 40 cm² to 180 cm², in particular of from 45 cm² to 170 cm², in particular of from 50 cm² to 160 cm², in particular of from 60 cm² to 150 cm², in particular of from 70 cm² to 140 cm², in particular of from 80 cm² to 130 cm², in particular of from 90 cm² to 120 cm², in particular of from 95 cm² to 110 cm².

In an embodiment, the first proximal cross-sectional area has a size lying in a range of from 0.5 cm² to 25 cm², in particular of from 1 cm² to 22.5 cm², in particular of from 2 cm² to 20 cm², in particular of from 3 cm² to 17.5 cm², in particular of from 4 cm² to 16 cm², in particular of from 5 cm² to 15 cm², in particular of from 6 cm² to 14 cm², in particular of from 7 cm² to 13 cm², in particular of from 8 cm² to 12 cm², in particular of from 9 cm² to 11 cm², in particular of from 9.5 cm² to 10 cm².

The diffusor zone has side walls that diverge in a direction from the proximal end of the diffusor zone towards the distal end of the diffusor zone at least in a section between the proximal end of the diffusor zone and the distal end of the diffusor zone, in particular over the whole length between the proximal end of the diffusor zone and the distal end of the diffusor zone. This divergence of the side walls results in an angle between the side walls lying in a range of from 5° to 30°, in particular of from 7.5° to 25°, in particular of from 10° to 20°, in particular of from 12.5° to 17.5°, in particular of from 14° to 16°. This angle between the side walls is defined as the angle between a first line running along a first of the side walls and a second line running along a second of the side walls.

In an embodiment, the filter element comprises a filter material support, wherein the filter material is arranged in the filter material support. In this context, the filter material support and/or the filter material have a circular base area having a diameter lying in a range of from 30 mm to 160 mm, in particular of from 35 mm to 150 mm, in particular of from 40 mm to 140 mm, in particular of from 45 mm to 130 mm, in particular of from 50 mm to 120 mm, in particular of from 55 mm to 110 mm, in particular of from 60 mm to 100 mm, in particular of from 70 mm to 90 mm, in particular of from 75 mm to 80 mm.

In an embodiment, the filter element comprises a filter material support, wherein the filter material is arranged in the filter material support, wherein the filter material support further comprises a plurality of ribs arranged distally of the filter material and keeping the filter material within the filter material support. These ribs are typically arranged such that they guarantee a particularly appropriate breathing air release from the breathing tube through the filter element towards an environment as well as a breathing air entry from an environment of the breathing tube arrangement through the filter element into an interior of the breathing tube. The ribs further provide a mechanic protection of the filter material since they prevent a user of the breathing tube arrangement from touching the filter material with his or her fingers. At least, the ribs make it much more difficult to contact the filter material with the fingers so that the surface of the filter material remains longer intact than in case of an unprotected filter material.

In an embodiment, at least some of the ribs of the plurality of ribs lie upon corresponding fins. In this context, the filter material is clamped between the ribs and the fins. Such an arrangement allows for a particularly appropriate and precise placement of the filter material in the filter material support, wherein a smooth surface of the filter material is achieved. A smooth surface of the filter material serves for a high repeatability of measurements and reduces the filter-to-filter variability.

In an embodiment, the filter material is clamped in the filter material support (in particular clamped between the ribs and the corresponding fins) such that a circumferential edge area of the filter material is more proximally or more distally arranged than a central area of the filter material. Thus, the clamping induces a curvature or bending of the filter material. Due to this curvature or bending of the filter material, a particularly exact positioning of the filter material in the filter material support as well as a particularly smooth and standardized surface of the filter material is achieved.

In an embodiment, the circumferential edge area of the filter material is more proximally or more distally arranged than the central area of the filter material by a distance lying in a range of from 0.5 mm to 10 mm, in particular from 1 mm to 9 mm, in particular from 1.5 mm to 8 mm, in particular from 2 mm to 7 mm, in particular from 2.5 mm to 6 mm, in particular from 3 mm to 5 mm, in particular from 3.5 mm to 4 mm.

In an embodiment, an angle between two lines defines the proximal or distal offset of the circumferential edge area of the filter material with respect to the central area of the filter material. The first line is a line intersecting a center of the central area of the filter material on a proximal surface of the filter material and a first support point on which a proximal surface of the circumferential edge area abuts a support surface of the filter material support. The second line is a line extending from the first support point through an axis running through the center of the central area of the filter material along a longitudinal extension direction of the breathing tube arrangement to a second support point on which the proximal surface of the circumferential edge area abuts the support surface of the filter material support. The angle lies in a range of from 1° to 10°, in particular of from 2° to 9°, in particular of from 3° to 8°, in particular of from 4° to 7°, in particular of from 5° to 6°.

In an embodiment, the filter material support comprises a filter material support body and a filter material support cover. The filter material support cover is intended to be placed over the filter material support body so as to form - together with the filter material support body - the filter material support. The filter material support body or the filter material support cover comprises at least one cam. The respective other of the filter material support body and the filter material support cover comprises at least one notch. The notch is arranged and designed for receiving the at least one cam. By an interaction of the notch and the cam, a connection between the filter material support body and the filter material support cover is established.

In an embodiment, the filter material support body and the filter material support cover comprise 2 to 10, in particular 3 to 9, in particular 4 to 8, in particular 5 to 7 cams and corresponding notches. In an embodiment, the cams and notches are equally distributed around the circumference of the filter material support body and the filter material support cover. Then, an x-fold symmetry is established, wherein x corresponds to the number of cams or notches. To give an example, if seven cams and seven notches are equally distributed around the circumference of the filter material support body and the filter material support cover, each of the filter material support body and the filter material support cover has a 7-fold symmetry so that both elements can be placed relatively to each other in seven defined positions. In an embodiment, the ribs (and optionally also the fins) are also arranged in an x-fold symmetry (wherein x corresponds once again to the number of cams or notches), e.g., in a 7-fold symmetry. Then, it will make no difference in which of the seven positions the filter material support cover is placed upon the filter material support body to clamp the filter material between the filter material support cover and the filter material support body. Rather, each of the possible positions will result in exactly the same final arrangement of the filter material support.

In an embodiment, the filter material, in particular a filtering layer of the filter material, comprises blended synthetic fibers.

In an embodiment, the filter material comprises a filtering layer (in particular a filtering layer comprising blended synthetic fibers) and at least one top layer laminated onto the filtering layer. The top layer can be laminated on one side of the filtering layer or on both sides of the filtering layer (i.e., on the sides through which the breathing air enters and exits the filter material upon passing through the filter material). If the top layer is only laminated on one side of the filtering layer, it is, in an embodiment, laminated to the proximal side of the filtering layer. Then, the top layer can particularly appropriate protect a subject using the breathing tube arrangement from inhaling the fine fibers of the filtering layer. The top layer serves for particularly smooth surface of the filter material so that the overall reliability of measurements performed with the breathing tube arrangement is increased (due to a reduced filter-to-filter variability and an increased repeatability of individual measurements).

In an embodiment, the top layer is a scrim layer. Scrim is a particularly appropriate material for achieving a smooth surface of the filter material, while not significantly increasing the breathing resistance of the filter material.

In an embodiment, the filter material, in particular a filtering layer of the filter material, is an electrostatic material. Spunbond polypropylene is a particularly appropriate filter material or a particularly appropriate material for the filtering layer.

Herewith disclosed but not forming part of the claimed invention is a filter element for a breathing tube of a breathing tube arrangement according to the preceding explanations. This filter element has a proximal opening (or inlet) that can be connected to a distal end of a breathing tube. Furthermore, a distal end of a breathing air releasing region of the filter element does not comprise a connector for connecting further elements of a breathing gas flow path to the breathing gas releasing region. Thus, this filter element distinguishes itself from prior art filter elements by not having a more or less symmetric design, but rather an asymmetric design without a connector element at its distal end. As explained above, refraining from providing a connector element on the distal side of the filter element significantly reduces the breathing resistance of the filter element, the material necessary for producing the filter element, as well as the dead space inside the filter element. These properties of the novel filter element synergistically act together with the flow properties of the filter element due to its diffusor zone and enhance the user-friendliness, measurement properties, and safety of a breathing tube equipped with such a filter element.

In an aspect, the present invention relates to a lung function diagnostics device according to claim 12 that comprises a breathing tube arrangement. According to this aspect of the invention, the breathing tube arrangement is inserted into the lung function diagnostics device such that a sensor of the lung function diagnostics device is able to analyze breathing air flowing through an analysis zone of a breathing tube of the breathing tube arrangement. It is furthermore inserted into the lung function diagnostics device such that a proximal end of the breathing tube is arranged proximally of the sensor of the lung function diagnostics device. Finally, the breathing tube arrangement is inserted into the lung function diagnostics device such that a filter element of the breathing tube arrangement is arranged distally of the sensor. In its final position in the lung function diagnostics device, the proximal end of the breathing tube is positioned on a first side of the lung function diagnostics device, whereas the distal end of the breathing tube arrangement (together with the filter element arranged at the distal end of the breathing tube arrangement) is located on a second side of the lung function diagnostics device.

In an embodiment, the sensor of the lung function diagnostics device is an ultrasonic sensor. An ultrasonic spirometer is a particularly appropriate lung function diagnostics device within the context of the presently claimed and described invention.

All embodiments of the breathing tube arrangement can be combined in any desired manner and can be transferred either individually or in any arbitrary combination to the described filter element and the described lung function diagnostics device. Likewise, all embodiments of the filter element can be combined in any desired manner and can be transferred either individually or in any arbitrary combination to the breathing tube arrangement and to the lung function diagnostics device. Finally, all embodiments of the lung function diagnostics device can be combined in any desired manner and can be transferred either individually or in any arbitrary combination to the breathing tube arrangement and to the filter element.

Further details of aspects of the present invention will be described in the following making reference to exemplary embodiments and accompanying Figures. In the Figures:
- Figure 1A: shows a perspective view of a first embodiment of a filter element;
- Figure 1B: shows a frontal view onto the distal end of the filter element of Figure 1A;
- Figure 1C: shows a longitudinal section through the filter element of Figure 1A along the line indicated with "C" in Figure 1B;
- Figure 1D: shows a perspective exploded view of the filter element of Figure 1A;
- Figure 1E: shows a detail of the filter material of the filter element of Figure 1A;
- Figure 2A: shows a perspective view of a second embodiment of a filter element;
- Figure 2B: shows a frontal view onto the distal end of the filter element of Figure 2A;
- Figure 2C: shows a longitudinal section through the filter element of Figure 2A along the line indicated with "C" in Figure 2B;
- Figure 2D: shows a longitudinal section through the filter element of Figure 2A along the line indicated with "D" in Figure 2B;
- Figure 3A: shows a breathing tube arrangement known from prior art;
- Figure 3B: shows a graphic depiction of the breathing resistance over the flow rate of the breathing tube arrangement of Figure 3A;
- Figure 4A: shows an embodiment of a breathing tube arrangement comprising a distal filter element; and
- Figure 4B: shows a graphic depiction of the breathing resistance over the flow rate of the breathing tube arrangement of Figure 4A.

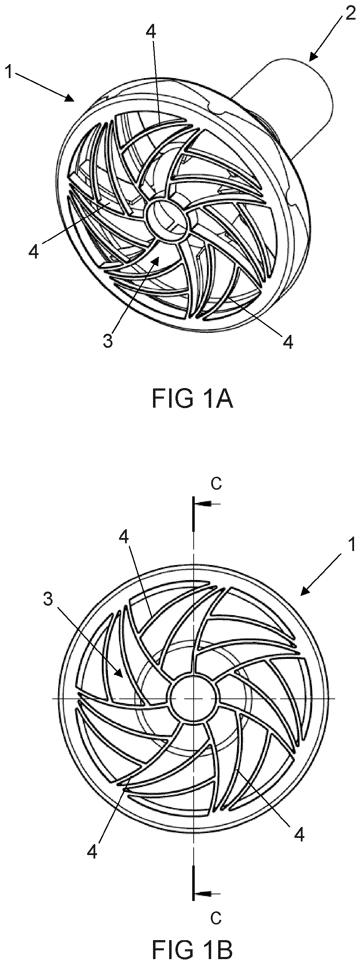

Figure 1A shows an embodiment of a filter element 1 comprising an inlet 2 at its proximal end and an outlet 3 at its distal end. A plurality of ribs 4 is provided for holding a filter material (not shown in Figure 1A) in place so that breathing air entering the filter element 1 through the inlet 2 and exiting the filter element 1 through the outlet 3 has to pass the filter material. For illustration purposes, only some of the ribs 4 are marked with the according numeral reference.

Figure 1B shows a frontal view onto the outlet 3 of the filter element 1 of Figure 1A. In this and in all following Figures, similar elements will be denoted with the same numeral reference.

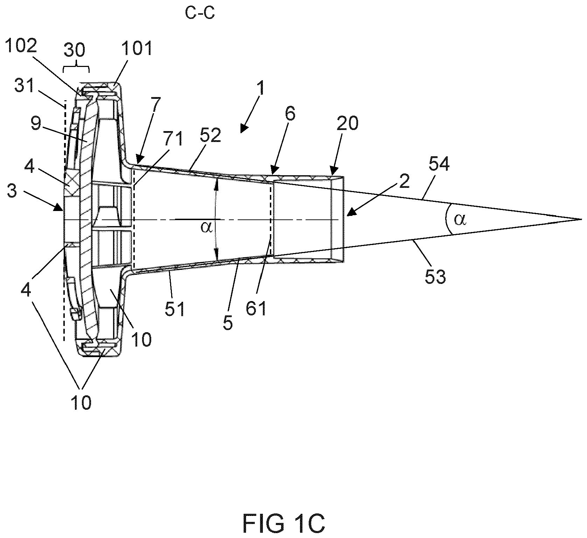

Figure 1C shows a longitudinal section through the filter element 1 of Figure 1A along the line indicated with "C" in Figure 1B. Here, it can well be seen that the inlet 2 is proximal of a diffusor 5 having a proximal end 6 and a distal end 7. The inlet 2 is realized at a connecting element 20 that is arranged proximal to the proximal end 6 of the diffusor 5. The connecting element 20 is intended to be connected to a distal end of a breathing tube. Upon such connection, the distal end of the breathing tube will be inserted into the proximal end region of the connecting element 20 up to a stop. An interior cross-section of the connecting element 20 is shaped such that no diameter offset between an interior of the distal end of the breathing tube and an interior of the proximal end 6 of the diffusor 5 is formed. Rather, the interiors of the distal end of the breathing tube and of proximal end 6 of the diffusor 5 will be flush. This reduces the risk of the occurrence of vortices and allows a laminar flow of gas through the breathing tube and the filter element 1.

Breathing air flowing through the breathing tube and further through the filter element 1 will then be guided through an interior of the diffusor 5 and through a filter material 9 that is kept in place by a filter material support 10. The ribs 4 form part of this filter material support 10.

A first side wall 51 and a second side wall 52 of the diffusor 5 diverge from each other in a continuous manner. A first line 53 extending along the first side wall 51 and a second line 54 extending along the second side wall 52 intersect each other at an angle α lying at approximately 12.5°.

Due to the diverging side walls 51, 52, the interior of the diffusor 5 acts as expansion. This design facilitates flowing of breathing air towards and through the filter material 9 and finally exiting the filter element 1 through the outlet 3.

The diverging side walls 51, 52 also cause that a proximal cross-sectional area 61 (corresponding to a first proximal cross-sectional area) is smaller than a distal cross-sectional area 71 (corresponding to a first distal cross-sectional area).

The filter element 1 does not comprise any connector on its distal end side (i.e., on the side of the outlet 3). Rather, the connecting element 20 is the only connector of the filter element 1. Therefore, it is not necessary that breathing air that has passed the filter material 9 needs again to flow through a connector having a comparatively small diameter. Rather, the whole area of the outlet 3 can be used by the breathing air upon exiting the filter element 1. The outlet 3 forms part of a breathing air releasing region 30, a cross-sectional area 31 of which is significantly larger than the proximal cross-sectional area 61 and the distal cross-sectional area 71 of the diffusor 5.

The sectional view of Figure 1C illustrates that the filter element 1 has a particularly small dead space. Many lung function measuring standards require small dead spaces so that the filter element 1 is appropriate to be used for many different measurements according to such standards. Furthermore, the analysis of breathing gas of children requires particularly small dead spaces since otherwise the breathing of the children would be changed.

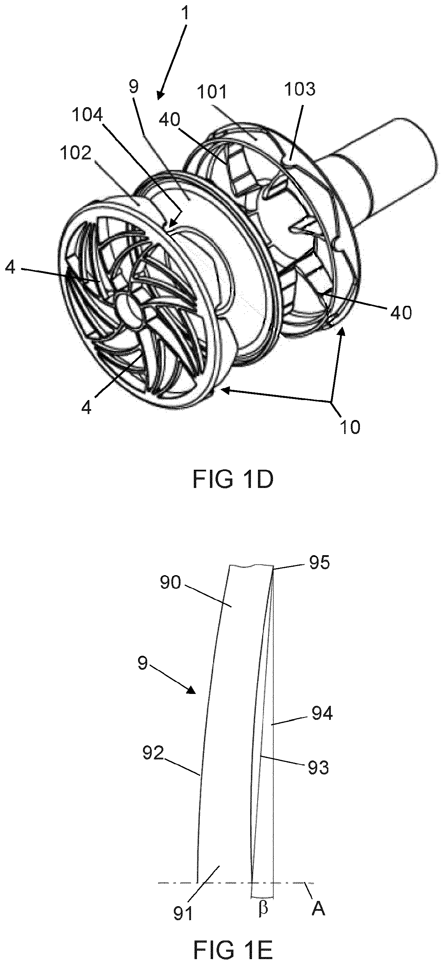

The filter material support 10 comprises a filter material support base 101 and a filter material support cover 102. The filter material support base 101 and the filter material support cover 102 receive the filter material 9 between them. This will be illustrated in Figure 1D in more detail.

Figure 1D shows an exploded perspective view of the filter element 1 already shown in Figures 1A to 1C. In this depiction, it can well be seen that the filter material support base 101 and the filter material support cover 102 form the filter material support 10. The ribs 4 are formed in the filter material support cover 102, whereas at least for some of the ribs 4 corresponding fins 40 are formed within the filter material support base 101. When the filter material 9 is placed between the filter material support base 101 and the filter material support cover 102, only those seven supporting ribs 4 that directly extend from the circular center rib 4 to the circumference of the filter material support cover 102 will abut the filter material 9. These seven supporting ribs 4 are supported by correspondingly arranged fins 40 in the filter material support base 101. Thus, the filter material 9 will be clamped between the seven supporting ribs 4 and the corresponding seven fins 40. The other ribs 4 in the filter material support cover 102 do not directly abut the filter material 9, but increase the mechanic stability of the filter material cover 102 and reduce the possibility for a user to contact the filter material 9 with her or his fingers.

In order to achieve an alignment of the seven supporting ribs 4 and the corresponding seven fins 40, the filter material support cover 102 needs to be placed upon the filter material support base 101 in a specific orientation. To particularly easily achieve this orientation, the filter material support base 101 comprises seven cams 103 (only one of these cams 103 is marked with the respective numeral reference), and the filter material support cover 102 comprises seven notches 104 (also here, only one of these notches 104 is marked with the respective numeral reference). Each of these notches 104 is intended to receive a corresponding cam 103. Once all cams 103 are received by corresponding notches 104, the filter material support cover 102 is tightly connected to the filter material support base 101.

Due to an equal distribution of the cams 103 and the notches 104, the filter material support cover 102 can be connected with the filter material support base 101 in seven different positions. Due to the symmetric design of the filter material support base 101 and the filter material support cover 102, each of these seven positions will result in the same final design of the filter material support 10.

Figure 1E shows a detail of the filter material 9 of the filter element 1 illustrated in Figures 1A to 1D. Here, a proximal offset of a circumferential edge area 90 of the filter material 9 with respect to a central area 91 of the filter material 9 is visible. This proximal offset is achieved by clamping the filter material 9 between the filter material support base 101 and the filter material support cover 102 (cf. Figure 1D). Due to this proximal offset, a surface 92 of the filter material 9 becomes particularly flat and smooth, thus increasing the reliability and repeatability of measurements performed when using the filter element 1.

The proximal offset can be defined by an angle between a first line 93 and a second line 94. The first line 93 intersects a proximal surface of the central area 91 of the filter material 9 at a central axis A running through a center of the central area 91 along a longitudinal direction of extension of the filter element 1 (and thus of a longitudinal direction of extension of the breathing tube arrangement comprising this filter element 1). The first line 93 further intersects a first support point 95 at which a proximal surface of the circumferential edge area 90 abuts a part of the filter material support. The second line 94 runs from this first support point 95 through the central axis a to a second support point lying opposite to the first support point 95 at the most distant part of the circumferential edge area 90 of the filter material support 9. The second support point is not shown in Figure 1E. An angle β between the first line 93 and the second line 94 has a value of approximately 4° in the embodiment shown in Figure 1E.

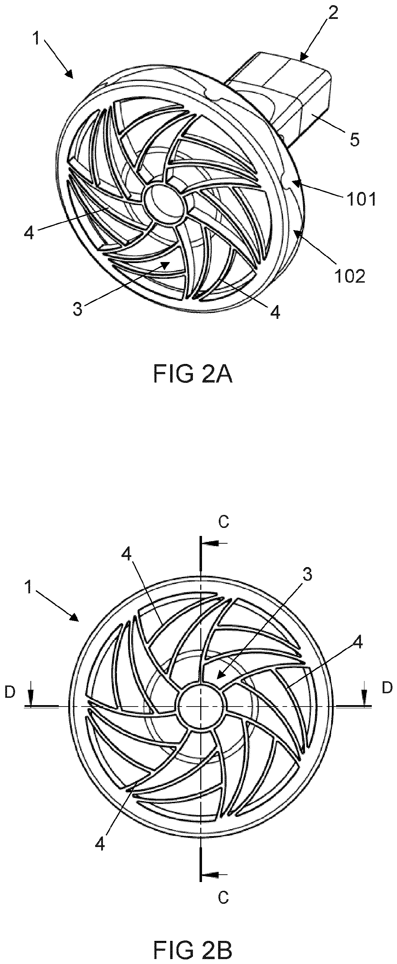

Figure 2A shows a perspective view of another embodiment of a filter element 1 that has a very similar design like the filter element shown in Figures 1A to 1C. However, the diffusor 5 surrounding the inlet 2 has a substantially rectangular cross section, whereas the cross-section of the diffusor 5 of the filter element of Figures 1A to 1D has a cylindrical cross-section. The other elements of the filter element 1 are highly similar or identical to the corresponding elements of the filter element shown in Figures 1A to 1C so that reference is made to the above given explanations.

Figure 2B shows a front view on the outlet 3 of the filter element 1 of Figure 2A. Also here, reference is made to the explanations given above with respect Figure 1B.

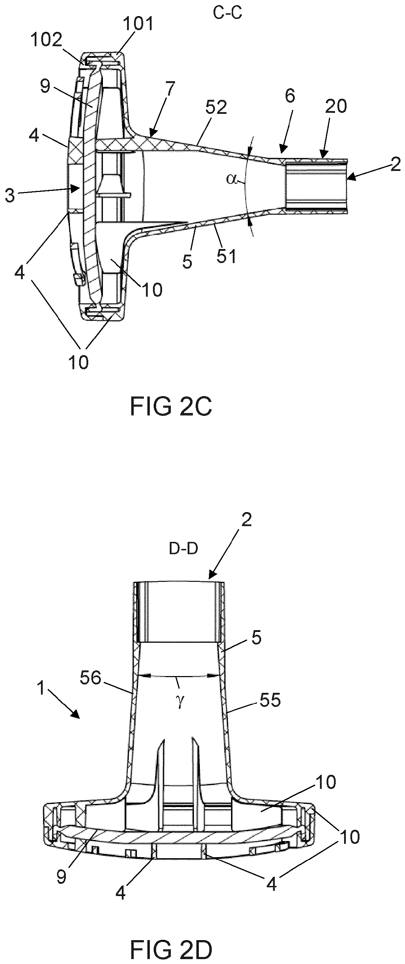

Figures 2C and 2D show longitudinal sections along the lines indicated with "C" or "D", respectively, in Figure 2B.

Due to the rectangular cross section of the diffusor 5, the longitudinal sections of Figure 2C and Figure 2D deviate from each other. The most prominent difference is a different angle between opposite side walls. Whereas a first side wall 51 and a second side wall 52 diverge at an angle α of approximately 20°, a third side wall 55 and a fourth side wall 56, as shown in Figure 2D, diverge at an angle γ of approximately 8°. However, even though the opening angle between the opposing sidewalls differs, the diffusor 5 still has the design of a diffusor or expansion. As a result, air being introduced through the inlet 2 into the filter element 1 can be easily flow towards and through the filter material 9 experiencing only a very low breathing resistance. Thus, the sidewalls 51, 52, 55, 56 diverging from the proximal end 6 to the distal end 7 of the diffusor 2 together with the big area of the outlet 3 serve for a particularly low respiratory resistance of the filter element 1.

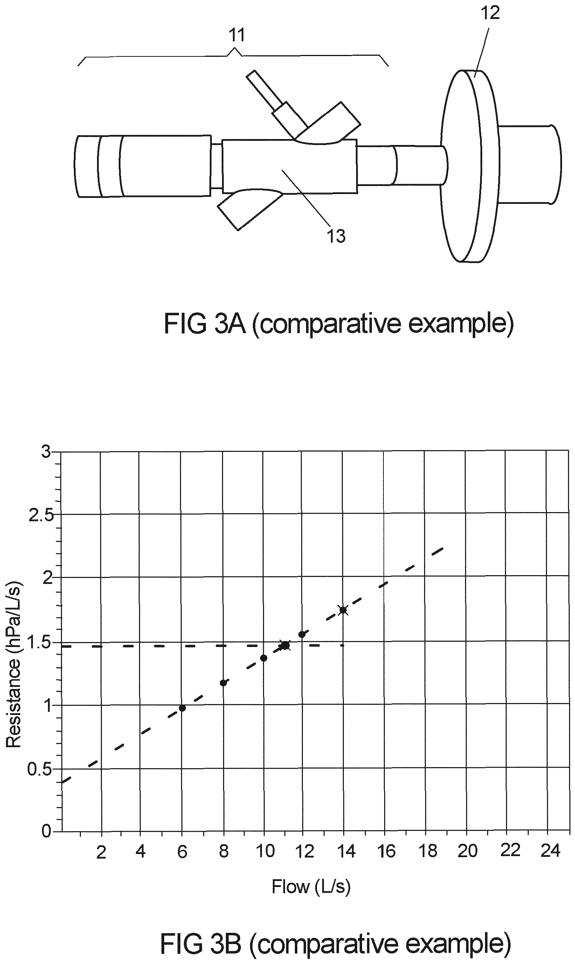

Figure 3A shows a breathing tube arrangement not falling under the claimed invention, but serving as comparative example. This breathing tube arrangement comprises a breathing tube 11 and a filter 12 located distally of an analysis zone 13 of the breathing tube 11. The filter 12 has an active filter area of approximately 60 cm². However, it does not comprise a diffusor zone. Furthermore, it has an outlet having a smaller area then the area of the filter element.

Figure 3B indicates the evolution of the breathing resistance with increasing flow of the breathing tube arrangement of Figure 3A. The maximum desired breathing resistance of 1.5 cm H₂O/L/s (corresponding to approximately 1.5 hPa/L/s) to comply with the ATS/ERS guidelines is indicated as reference value (dashed horizontal curve). It can be clearly seen from Figure 3B that the breathing resistance (individual measuring points fitted with the dashed ascending curve) linearly increases with increasing flow. The recommended maximum breathing resistance is already reached at a flow of approximately 11 L/s. At a flow of 14 L/s, the breathing resistance has reached a value of approximately 1.75 hPa/L/s and thus lies significantly over the recommended maximum breathing resistance. Thus, this comparative breathing tube arrangement is not able to comply with the recommended breathing resistance.

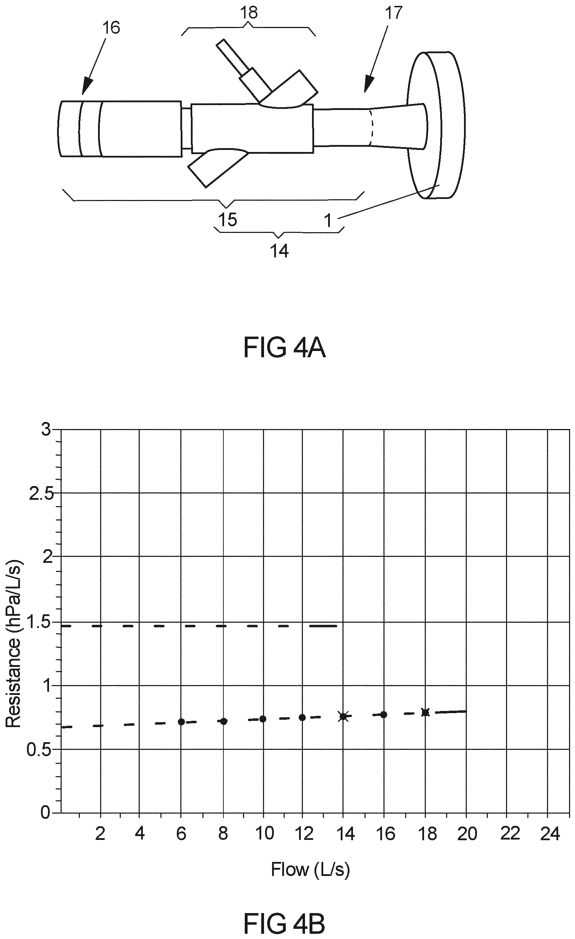

Figure 4A shows a breathing tube arrangement 14 comprising a breathing tube 15 and a filter element 1 with a diffusor zone. The breathing tube 15 comprises a proximal end 16 and a distal end 17 as well as an analysis zone 18 located between the proximal end 16 and the distal end 17. The filter element 1 is the same filter element as illustrated in more detail in Figures 1A to 1D. It is located at the distal end 17 of the breathing tube 15 and has an active filter area of approximately 45 cm².

Figure 4B shows the breathing resistance of the breathing tube arrangement 14 of Figure 4A over the flow rate. The same filter material as in the setup of the comparative example of Figure 3A was used. Once again, the recommended maximum breathing resistance of approximately 1.5 hPa/L/s is indicated as reference value (dashed horizontal curve). Here, it can clearly be seen that the breathing resistance of the breathing tube arrangement 14 with the distally positioned filter element 1 having a diffusor zone exhibits a breathing resistance that is significantly lower than the maximum recommended breathing resistance (individual measuring points fitted with the dashed ascending curve). At a flow rate of 14 L/s, the breathing resistance is approximately 0.75 hPa/L/s and thus less than half of the breathing resistance of the comparative breathing tube arrangement depicted in Figure 3A (cf. also the experimental results of Figure 3B). This clearly shows the superiority of a distally positioned filter element having a diffusor zone like in case of the breathing tube arrangement 14 shown in Figure 4A.

## Claims

1. Breathing tube arrangement for a lung function diagnostics device, comprising a breathing tube (15) defining a guiding path for breathing air to be analyzed by a lung function diagnostics device, the breathing tube (15) having a proximal end (16), a distal end (17) and an analysis zone (18) located between the proximal (16) end and the distal end (17), wherein the proximal end (16) is designed to face a subject whose lung function is to be analyzed and to receive exhaled breathing air from the subject, wherein the analysis zone (18) serves for allowing an analysis of breathing air flowing through the breathing tube (15), and wherein the distal end (17) serves for releasing analyzed breathing air to an environment of the breathing tube,
wherein the breathing tube arrangement (14) comprises a filter element (1) having a filter material (9), a breathing air releasing region (30) arranged distally of the filter material (9), and a diffusor zone (5) arranged proximally of the filter material (9),
wherein the diffusor zone (5) comprises a proximal end (6) having a first proximal cross-sectional area (61) and a distal end (7) having a first distal cross-sectional area (71) that is bigger than the first proximal cross-sectional area (61), wherein the proximal end (6) of the diffusor zone (5) is connected to the distal end (17) of the breathing tube (15) such that breathing air flowing from the breathing tube (15) through the distal end (17) of the breathing tube (15) towards an environment of the breathing tube (15) has to pass the filter element (1), and
wherein the breathing air releasing region (30) has a second distal cross-sectional area (31), wherein the second distal cross-sectional area (31) is bigger than the first proximal cross-sectional area (61) and at least as big as the first distal cross-sectional area (71), wherein a ratio between the first proximal cross-sectional area (61) and the second distal cross-sectional area (31) lies in a range from 1:2 to 1:20,
**characterized**
**in that** the diffusor zone (5) has sidewalls (51, 52, 55, 56) that diverge in a direction from the proximal end (6) of the diffusor zone (5) towards the distal end (7) of the diffusor zone (5) at least in a section between the proximal end (6) of the diffusor zone (5) and the distal end (7) of the diffusor zone (5) at an angle lying in a range from 5° to 30°.

2. Breathing tube arrangement according to claim 1, **characterized in that** a distal end (3) of the breathing air releasing region (30) does not comprise a connector for connecting further elements of a breathing gas flow path to the breathing gas releasing region (30).

3. Breathing tube arrangement according to claim 1 or 2, **characterized in that** the filter material (9) is arranged in a filter material support (10), wherein the filter material support (10) and/or the filter material (9) have a circular base area having a diameter lying in a range from 30 mm to 160 mm.

4. Breathing tube arrangement according to any of the preceding claims, **characterized in that** the filter material (9) is arranged in a filter material support (10), wherein the filter material support (10) further comprises a plurality of ribs (4) arranged distally of the filter material (9) and keeping the filter material (9) within the filter material support (10).

5. Breathing tube arrangement according to claim 4, **characterized in that** at least some ribs of the plurality of ribs (4) lie upon corresponding fins (40), wherein the filter material (9) is clamped between the ribs (4) and the fins (40).

6. Breathing tube arrangement according to any of claims 3 to 5, **characterized in that** the filter material (9) is clamped in the filter material support (10) such that a circumferential edge area (90) of the filter material (9) is more proximally or more distally arranged than a central area (91) of the filter material (9).

7. Breathing tube arrangement according to claim 6, **characterized in that** an angle between i) a first line (93) intersecting a center of the central area (92) of the filter material (9) on a proximal surface of the filter material (9) and a first support point (95) on which a proximal surface of the circumferential edge area (90) abuts a support surface of the filter material support (10) and ii) a second line (94) extending from the first support point (95) through an axis (A) running through the center of the central area (91) of the filter material (9) along a longitudinal extension direction of the breathing tube arrangement (14) to a second support point on which the proximal surface of the circumferential edge area (90) abuts the support surface of the filter material support (10) lies in a range from 1° to 10°.

8. Breathing tube arrangement according to any of claims 3 to 7, **characterized in that** the filter material support (10) comprises a filter material support body (101) and a filter material support cover (102), wherein one of the filter material support body (101) and the filter material support cover (102) comprises at least one cam (103) and the other of the filter material support body (101) and the filter material support cover (102) comprises at least one notch (104) for receiving the at least one cam (103).

9. Breathing tube arrangement according to any of the preceding claims, **characterized in that** the filter material (9) comprising a filtering layer comprising blended synthetic fibers.

10. Breathing tube arrangement according to any of the preceding claims, **characterized in that** the filter material (9) comprises a filtering layer and a top layer laminated onto the filtering layer.

11. Breathing tube arrangement according to claim 10, **characterized in that** the top layer is a scrim layer.

12. Lung function diagnostics device comprising a breathing tube arrangement (14) according to any of claims 1 to 9, **characterized in that** the breathing tube arrangement (14) is inserted into the lung function diagnostics device such that i) a sensor of the lung function diagnostics device is able to analyze breathing air flowing through an analysis zone (18) of a breathing tube (15) of the breathing tube arrangement (14), ii) a proximal end (16) of the breathing tube (15) is arranged proximally of the sensor, and iii) a filter element (1) of the breathing tube arrangement (14) is arranged distally of the sensor.

13. Lung function diagnostics device according to claim 12, **characterized in that** the sensor is an ultrasonic sensor.

## Patentansprüche

1. Atemrohranordnung für ein Lungenfunktionsdiagnosegerät, mit einem Atemrohr (15), das einen Führungsweg für durch ein Lungenfunktionsdiagnosegerät zu analysierende Atemluft definiert, wobei das Atemrohr (15) ein proximales Ende (16), ein distales Ende (17) und eine zwischen dem proximalen Ende (16) und dem distalen Ende (17) befindliche Analysezone (18) besitzt, wobei das proximale Ende (16) derart ausgebildet ist, dass es einem Patienten, dessen Lungenfunktion zu analysieren ist, zugewandt ist und dazu ausgebildet ist, ausgeatmete Atemluft von dem Patienten aufzunehmen, wobei die Analysezone (18) dazu dient, eine Analyse der durch das Atemrohr (15) strömenden Atemluft zu erlauben, und wobei das distale Ende (17) dazu dient, analysierte Atemluft in eine Umgebung des Atemrohrs freizusetzen,
wobei die Atemrohranordnung (14) ein Filterelement (1) mit einem Filtermaterial (9), einen distal von dem Filtermaterial (9) angeordneten Atemluftfreisetzungsbereich (30) und eine proximal von dem Filtermaterial (9) angeordnete Diffusorzone (5) umfasst,
wobei die Diffusorzone (5) ein proximales Ende (6) mit einer ersten proximalen Querschnittsfläche (61) und ein distales Ende (7) mit einer ersten distalen Querschnittsfläche (71) umfasst, die größer ist als die erste proximale Querschnittsfläche (61), wobei das proximale Ende (6) der Diffusorzone (5) mit dem distalen Ende (17) des Atemrohres (15) so verbunden ist, dass von dem Atemrohr (15) durch das distale Ende (17) des Atemrohres (15) in Richtung einer Umgebung des Atemrohres (15) strömende Atemluft das Filterelement (1) passieren muss, und
wobei der Atemluftfreisetzungsbereich (30) eine zweite distale Querschnittsfläche (31) besitzt, wobei die zweite distale Querschnittsfläche (31) größer ist als die erste proximale Querschnittsfläche (61) und mindestens so groß wie die erste distale Querschnittsfläche (71), wobei ein Verhältnis zwischen der ersten proximalen Querschnittsfläche (61) und der zweiten distalen Querschnittsfläche (31) in einem Bereich von 1:2 bis 1:20 liegt,
**dadurch gekennzeichnet,**
**dass** die Diffusorzone (5) Seitenwände (51, 52, 55, 56) besitzt, die in einer Richtung von dem proximalen Ende (6) der Diffusorzone (5) hin zu dem distalen Ende (7) der Diffusorzone (5) zumindest in einem Abschnitt zwischen dem proximalen Ende (6) der Diffusorzone (5) und dem distalen Ende (7) der Diffusorzone (5) in einem im Bereich von 5° bis 30° liegenden Winkel auseinanderlaufen.

2. Atemrohranordnung nach Anspruch 1, **dadurch gekennzeichnet, dass** ein distales Ende (3) des Atemluftfreisetzungsbereichs (30) kein Verbindungsstück zum Verbinden weiterer Elemente eines Atemgasströmungswegs mit dem Atemgasfreisetzungsbereich (30) umfasst.

3. Atemrohranordnung nach Anspruch 1 oder 2, **dadurch gekennzeichnet, dass** das Filtermaterial (9) in einem Filtermaterialträger (10) angeordnet ist, wobei der Filtermaterialträger (10) und/oder das Filtermaterial (9) eine kreisförmige Grundfläche mit einem im Bereich von 30 mm bis 160 mm liegenden Durchmesser besitzen.

4. Atemrohranordnung nach einem der vorhergehenden Ansprüche, **dadurch gekennzeichnet, dass** das Filtermaterial (9) in einem Filtermaterialträger (10) angeordnet ist, wobei der Filtermaterialträger (10) ferner eine Vielzahl von Rippen (4) umfasst, die distal von dem Filtermaterial (9) angeordnet sind und das Filtermaterial (9) in dem Filtermaterialträger (10) halten.

5. Atemrohranordnung nach Anspruch 4, **dadurch gekennzeichnet, dass** wenigstens einige Rippen der Vielzahl von Rippen (4) auf entsprechenden Stegen (40) liegen, wobei das Filtermaterial (9) zwischen den Rippen (4) und den Stegen (40) eingeklemmt ist.

6. Atemrohranordnung nach einem der Ansprüche 3 bis 5, **dadurch gekennzeichnet, dass** das Filtermaterial (9) so in dem Filtermaterialträger (10) eingeklemmt ist, dass ein Umfangskantenbereich (90) des Filtermaterials (9) proximaler oder distaler angeordnet ist als ein mittlerer Bereich (91) des Filtermaterials (9).

7. Atemrohranordnung nach Anspruch 6, **dadurch gekennzeichnet, dass** ein Winkel zwischen i) einer ersten Linie (93), die einen Mittelpunkt des mittleren Bereichs (92) des Filtermaterials (9) auf einer proximalen Fläche des Filtermaterials (9) und einen ersten Stützpunkt (95), an dem eine proximale Fläche des Umfangskantenbereichs (90) gegen eine Auflagefläche des Filtermaterialträgers (10) stößt, schneidet, und ii) einer zweiten Linie (94), die sich von dem ersten Stützpunkt (95) durch eine durch den Mittelpunkt des mittleren Bereichs (91) des Filtermaterials (9) entlang einer Längserstreckungsrichtung der Atemrohranordnung (14) verlaufende Achse (A) zu einem zweiten Stützpunkt erstreckt, an dem die proximale Fläche des Umfangskantenbereichs (90) gegen die Auflagefläche des Filtermaterialträgers (10) stößt, in einem Bereich von 1° bis 10° liegt.

8. Atemrohranordnung nach einem der Ansprüche 3 bis 7, **dadurch gekennzeichnet, dass** der Filtermaterialträger (10) einen Filtermaterialträgerkörper (101) und eine Filtermaterialträgerabdeckung (102) umfasst, wobei eines von dem Filtermaterialträgerkörper (101) und der Filtermaterialträgerabdeckung (102) mindestens einen Nocken (103) umfasst und das andere von dem Filtermaterialträgerkörper (101) und der Filtermaterialträgerabdeckung (102) mindestens eine Kerbe (104) zur Aufnahme des mindestens einen Nockens (103) umfasst.

9. Atemrohranordnung nach einem der vorhergehenden Ansprüche, **dadurch gekennzeichnet, dass** das eine Filterschicht umfassende Filtermaterial (9) gemischte Kunstfasern umfasst.

10. Atemrohranordnung nach einem der vorhergehenden Ansprüche, **dadurch gekennzeichnet, dass** das Filtermaterial (9) eine Filterschicht und eine auf die Filterschicht kaschierte Deckschicht umfasst.

11. Atemrohranordnung nach Anspruch 10, **dadurch gekennzeichnet, dass** die Deckschicht eine Gazeschicht ist.

12. Lungenfunktionsdiagnosegerät mit einer Atemrohranordnung (14) nach einem der Ansprüche 1 bis 9, **dadurch gekennzeichnet, dass** die Atemrohranordnung (14) so in das Lungenfunktionsdiagnosegerät eingesetzt ist, dass i) ein Sensor des Lungenfunktionsdiagnosegeräts in der Lage ist, durch eine Analysezone (18) eines Atemrohres (15) der Atemrohranordnung (14) strömende Atemluft zu analysieren, ii) ein proximales Ende (16) des Atemrohres (15) proximal von dem Sensor angeordnet ist, und iii) ein Filterelement (1) der Atemrohranordnung (14) distal von dem Sensor angeordnet ist.

13. Lungenfunktionsdiagnosegerät nach Anspruch 12, **dadurch gekennzeichnet, dass** der Sensor ein Ultraschallsensor ist.

## Revendications

1. Agencement de tube respiratoire pour un dispositif de diagnostic de la fonction pulmonaire, comprenant un tube respiratoire (15) définissant un chemin de guidage pour l'air respiratoire devant être analysé par un dispositif de diagnostic de la fonction pulmonaire, le tube respiratoire (15) ayant une extrémité proximale (16), une extrémité distale (17) et une zone d'analyse (18) située entre l'extrémité proximale (16) et l'extrémité distale (17), dans lequel l'extrémité proximale (16) est conçue pour faire face à un sujet dont la fonction pulmonaire doit être analysée et pour recevoir l'air respirable expiré par le sujet, dans lequel la zone d'analyse (18) sert à permettre une analyse de l'air respirable circulant dans le tube respiratoire (15), et dans lequel l'extrémité distale (17) sert à libérer l'air respirable analysé dans l'environnement du tube respiratoire,
dans lequel l'agencement de tube respiratoire (14) comprend un élément filtrant (1) avec un matériau filtrant (9), une zone de libération de l'air respirable (30) agencée distalement par rapport au matériau filtrant (9), et une zone de diffusion (5) agencée proximalement par rapport au matériau filtrant (9),
dans lequel la zone de diffusion (5) comprend une extrémité proximale (6) ayant une première surface de section transversale proximale (61) et une extrémité distale (7) ayant une première surface de section transversale distale (71) plus grande que la première surface de section transversale proximale (61), dans lequel l'extrémité proximale (6) de la zone de diffusion (5) est reliée à l'extrémité distale (17) du tube respiratoire (15) de sorte que l'air respirable s'écoulant du tube respiratoire (15) à travers l'extrémité distale (17) du tube respiratoire (15) vers un environnement du tube respiratoire (15) doit passer l'élément filtrant (1), et
dans lequel la zone de libération de l'air respirable (30) présente une deuxième surface de section transversale distale (31), la deuxième surface de section transversale distale (31) étant plus grande que la première surface de section transversale proximale (61) et au moins aussi grande que la première surface de section transversale distale (71), un rapport entre la première surface de section transversale proximale (61) et la deuxième surface de section transversale distale (31) étant compris entre 1:2 et 1:20,
**caractérisé**
**en ce que** la zone de diffusion (5) possède des parois latérales (51, 52, 55, 56) qui divergent dans une direction de l'extrémité proximale (6) de la zone de diffusion (5) vers l'extrémité distale (7) de la zone de diffusion (5) au moins dans une section entre l'extrémité proximale (6) de la zone de diffusion (5) et l'extrémité distale (7) de la zone de diffusion (5) à un angle compris entre 5° et 30°.

2. Agencement de tube respiratoire selon la revendication 1, **caractérisé en ce qu'**une extrémité distale (3) de la zone de libération de l'air respirable (30) ne comprend pas de connecteur pour relier d'autres éléments d'un trajet d'écoulement de gaz respirable à la zone de libération de gaz respirable (30).

3. Agencement de tube respiratoire selon la revendication 1 ou 2, **caractérisé en ce que** le matériau filtrant (9) est agencé dans un support de matériau filtrant (10), dans lequel le support de matériau filtrant (10) et/ou le matériau filtrant (9) présentent une surface de base circulaire dont le diamètre est compris entre 30 mm et 160 mm.

4. Agencement de tube respiratoire selon l'une quelconque des revendications précédentes, **caractérisé en ce que** le matériau filtrant (9) est agencé dans un support de matériau filtrant (10), dans lequel le support de matériau filtrant (10) comprend en outre une pluralité de nervures (4) agencées distalement par rapport au matériau filtrant (9) et maintenant le matériau filtrant (9) à l'intérieur du support de matériau filtrant (10).

5. Agencement de tube respiratoire selon la revendication 4, **caractérisé en ce qu'**au moins certaines nervures de la pluralité de nervures (4) reposent sur des ailettes correspondantes (40), le matériau filtrant (9) étant serré entre les nervures (4) et les ailettes (40).

6. Agencement de tube respiratoire selon l'une quelconque des revendications 3 à 5, **caractérisé en ce que** le matériau filtrant (9) est serré dans le support de matériau filtrant (10) de telle sorte qu'une zone de bord circonférentiel (90) du matériau filtrant (9) est agencée plus proximalement ou plus distalement qu'une zone centrale (91) du matériau filtrant (9).

7. Agencement de tube respiratoire selon la revendication 6, **caractérisé en ce qu'**un angle entre i) une première ligne (93) coupant un centre de la zone centrale (92) du matériau filtrant (9) sur une surface proximale du matériau filtrant (9) et un premier point d'appui (95) sur lequel une surface proximale de la zone du bord circonférentiel (90) est en contact avec une surface d'appui du support de matériau filtrant (10) et ii) une deuxième ligne (94) s'étendant à partir du premier point d'appui (95) à travers un axe (A) passant par le centre de la zone centrale (91) du matériau filtrant (9) le long d'une direction d'extension longitudinale de l'agencement de tube respiratoire (14) jusqu'à un deuxième point d'appui sur lequel la surface proximale de la zone de bord circonférentiel (90) est en contact avec la surface d'appui du support de matériau filtrant (10) se situe dans une plage de 1° à 10°.

8. Agencement de tube respiratoire selon l'une quelconque des revendications 3 à 7, **caractérisé en ce que** le support de matériau filtrant (10) comprend un corps de support de matériau filtrant (101) et un couvercle de support de matériau filtrant (102), dans lequel l'un du corps de support de matériau filtrant (101) et le couvercle de support de matériau filtrant (102) comprennent au moins une came (103) et l'autre du corps de support de matériau filtrant (101) et le couvercle de support de matériau filtrant (102) comprennent au moins une encoche (104) pour recevoir l'au moins une came (103).

9. Agencement de tube respiratoire selon l'une quelconque des revendications précédentes, **caractérisé en ce que** le matériau filtrant (9) comprend une couche filtrante comportant de fibres synthétiques mélangées.

10. Agencement de tube respiratoire selon l'une quelconque des revendications précédentes, **caractérisé en ce que** le matériau filtrant (9) comprend une couche filtrante et une couche supérieure laminée sur la couche filtrante.

11. Agencement de tube respiratoire selon la revendication 10, **caractérisé en ce que** la couche supérieure est une couche de gaze.

12. Dispositif de diagnostic de la fonction pulmonaire comprenant un agencement de tube respiratoire (14) selon l'une quelconque des revendications 1 à 9, **caractérisé en ce que** l'agencement de tube respiratoire (14) est inséré dans le dispositif de diagnostic de la fonction pulmonaire de telle sorte que i) un capteur du dispositif de diagnostic de la fonction pulmonaire est capable d'analyser l'air respirable circulant à travers une zone d'analyse (18) d'un tube respiratoire (15) de l'agencement de tube respiratoire (14), ii) une extrémité proximale (16) du tube respiratoire (15) est agencé proximalement par rapport au capteur, et iii) un élément filtrant (1) de l'agencement de tube respiratoire (14) est agencé distalement par rapport au capteur.

13. Dispositif de diagnostic de la fonction pulmonaire selon la revendication 12, **caractérisé en ce que** le capteur est un capteur à ultrasons.
